# EUROPÄISCHE PATENTSCHRIFT

(11) **EP 0 582 186 B1**
(45) Veröffentlichungstag und Bekanntmachung des Hinweises auf die Patenterteilung: **24.02.1999**
(21) Anmeldenummer: 93112018.2
(22) Anmeldetag: 28.07.1993
(51) Int. Cl.: A61K 9/16, A61K 9/20, A61K 47/42

(54) **Verfahren zur Herstellung von festen Arzneiformkörpern mit protrahierter 2-Stufen-Freisetzung**
Process for producing solid pharmaceutical dosage forms with an extended two-stage release
Procédé de préparation de formes pharmaceutiques solides à libération prolongée en deux étapes

(30) Priorität: 04.08.1992 DE 4225730
(43) Veröffentlichungstag der Anmeldung: 09.02.1994
(73) Patentinhaber: MERZ + CO. GmbH & Co., 60318 Frankfurt (DE)
(72) Erfinder: Nürnberg, Eberhard, Prof. Dr., D-91080 Uttenreuth (DE); Seiller, Ehrhard, Dr., D-61130 Nidderau (DE); Ritsert, Stefan, D-69412 Eberbach (DE)
(74) Vertreter: Beil, Hans Chr., Dr.

(56) Entgegenhaltungen:
- EP-A- 0 357 401
- EP-A- 0 442 012
- EP-A- 0 447 100
- WO-A-91/06287

## Beschreibung

Die vorliegende Erfindung betrifft ein Verfahren zur Herstellung von festen Arzneiformkörpern mit protrahierter 2-Stufen-Freisetzung, welches gekennzeichnet ist durch den Zusatz eines wasserlöslichen sowie eines wasserunlöslichen Salzes von Casein, insbesondere von Natrium- und Calciumcaseinat, in einem breiten Verhältnis und in einer Gesamtmenge von 5-98%.

Das Verfahren ist u.a. geeignet zur Herstellung von Formkörpern, enthaltend Memantine als Wirkstoff.

Orale Arzneimittelformulierungen mit verzögerter Freisetzung, sogenannte Retardpräparate, sind Produkte mit einer Freistzung über einen längeren Zeitraum und somit einer verlängerten Wirkung, wobei die sich einstellenden Plasmaspiegel dem tatsächlichen Bedarf angepaßt sein sollen. Dies bedeutet jedoch nicht, daß stets konstante Blutspiegelkonzentrationen erreicht werden sollen. Vielmehr kann auch eine mehrphasige Freisetzung geeignet sein, um das gewünschte therapeutische Ziel zu erreichen. Ferner sollen systemische Nebenwirkungen und lokale unerwünschte Wirkungen im Gastrointestinaltrakt durch zu hohe lokale Konzentrationen bzw. Plasmaspiegel vermieden werden.

Bei den bisher bekannten Verfahren zur Herstellung von Arzneiformen mit verzögerter Freisetzung werden die Wirkstoffe meist durch Aufbringen von Überzügen behandelt oder in makromolekulare Substanzen eingebettet bzw. mit diesen zusammen verarbeitet.

Die wichtigsten Steuerungsprinzipien der Wirkstoff-Freisetzung aus Arzneiformkörpern sind das Film-coating und die Matrix. Beim Film-coating werden filmbildende Polymere eingesetzt, die den Wirkstoff diffusions-kontrolliert freigeben. Nachteilig wirkt sich diese Formulierung dann aus, wenn beim Zerteilen oder Zerkauen der oralen Arzneiform eine höhere Wirkstoffdosis freigesetzt wird, was zu unerwünschten Überdosierungserscheinungen führen kann.

Bei der Matrix-kontrollierten Freisetzung werden lipophile Substanzen, z.B. höhere Alkohole, Wachse, unlösliche Thermoplaste verwendet. Hierbei wirkt sich nachteilig aus, daß synthetische Polymere stets wechselnde Mengen an Monomeren enthalten und eine vollständige Freissetzung oftmals nicht erreicht werden kann.

In der US-A 4 665 081 wird eine Nifedipin-Formulierung zur oralen Applikation beschrieben, welche Casein und anorganische Zusätze, ausgewählt aus Magnesiumsilikat, -oxid, -aluminatmetasilikat, synthetischem Hydrotalk und Magnesiumaluminumoxid, enthält. Hiermit wird erreicht, daß der Wirkstoff, unter der Premisse, daß ein magensaftresistenter Hilfsstoff zugesetzt wird, im Magen nicht, im Darm jedoch sehr schnell freigesetzt wird. Dadurch wird einerseits zwar verzögert - im Bezug auf Verabreichungszeitpunkt - freigesetzt, andererseits jedoch durch die rasche Auflösung im Darm sehr schnell eine hohe Plasmakonzentration erreicht, was zu unerwünschten Nebenwirkungen führen kann.

In Pharm.Acta Helv., 66, No. 4, 1991, S.120-124, wird eine Ibuprofen-Formulierung beschrieben, die niedermolekulares Casein oder Gelatine enthält. Dies bewirkt eine erhöhte Auflösungs- bzw. Freisetzungsrate des Wirkstoffs.

In Pharmaceutical Technology, 9, 1990, S. 360-374, wird der Zusatz von Natriumcaseinat auf die Freisetzungsrate von Wirkstoffen untersucht. Auch hier wird eine erhöhte Auflössung, insbesondere von Chlorothiazid und Hydrochlorothiazid, erzielt.

In der EP-A 0 447 100 werden Formulierungen beschrieben, welche eine kontrollierte Freisetzung im Magen und im Darm in Abhängigkeit von den dort vorhandenen Enzymen zuläßt. Hierbei wird eine Gelmatrix, z.B. aus Alginat oder Carboxymethylcellulose, Carrageen u.ä., verwendet, die ein Protein, wie z.B. Calciumcaseinat, eingelagert enthält und einen weiteren Stoff aufweist, der an das Protein binden kann. Dies kann ein Arzneimittel oder ein Lebensmittel (Kaugummi) sein.

Zwar ist hiermit eine kontrollierte Freisetzung möglich, jedoch wird der Effekt durch die Inkorporation des Proteins in einem fremden Matrixbildner erreicht.

In der GB-A 2 207 353 werden ebenfalls Formulierungen mit kontrollierter Freisetzung beschrieben, enthaltend calciumfreie Gemische von Alginsäuresalzen und Caseinat. Die protrahierte Freisetzung beruht jedoch hier auf einem wie oben angegebenen Prinzip.

Aufgabe vorliegender Erfindung ist es, eine Formulierung für Arzneimittel mit einer kontrollierten Freisetzung bereitzustellen, derart, daß zumindest über 2 Stufen der Wirkstoff über einen längeren Zeitraum abgegeben werden kann.

Diese Aufgabe wird erfindungsgemäß dadurch gelöst, daß man einer Mischung, enthaltend einen oder mehrere Wirkstoffe sowie übliche Hilfs- und Zusatzstoffe, insgesamt 5-98%, bevorzugt 10-90% und insbesondere 30-80%, bezogen auf die Gesamtmenge, eines wasserlöslichen sowie eines wasserunlöslichen Salzes von Casein zusetzt, wobei die Caseinsalzmischung 5-95% des wasserunlöslichen Caseinats aufweist. Eine derartige Formulierung kann sodann auf übliche Weise behandelt werden . Hierzu zählen z.B. die in der pharmazeutischen Industrie bekannten Methoden der Kompression, Granulierung, Extrusion, Pelletisierung und Tablettierung auf trockenem oder feuchtem Weg. Es können auch verschiedene Methoden kombiniert werden, um die gewünschte Produktformulierung, wie z.B. Tablette, Dragee, Pellets, bereitzustellen. Die Wirkstoffmenge kann dabei je nach Indikation und Art in weiten Bereichen variieren, wie z.B. von 0,01 bis 90%, bezogen auf die Gesamtmenge.

Hierbei zeigte sich überraschenderweise, daß die pharmakokinetischen Eigenschaften der erfindungsgemäßen Formulierung von der jeweiligen Herstellungsmethode und hiermit verbundenen praxisgerechten Schwankungen, z.B. der Preßkräfte, nicht beeinträchtigt werden.

Als Casein kann ein handelsübliches Produkt verwendet werden, wobei dessen Molekulargewicht und Wassergehalt in weiten Bereichen, wie z.B. von MW 18000 bis MW 30000 Dalton - je nach Herkunft - schwanken kann. Casein ist eine lebensmittelrechtlich zugelassene Substanz.

Als wasserlösliches Salz wird ein Caseinsalz eines einwertigen Kations, wie z.B. Lithium, Kalium, Ammonium und vorzugsweise Natrium, verwendet.

Als wasserunlösliches Caseinat wird ein Salz des Caseins mit zwei- oder mehrwertigen Kationen eingesetzt. Hierzu zählen z.B. Calcium, Magnesium, Zink, Mangan, Aluminium, Eisen oder Mischungen hiervon. Insbesondere bevorzugt ist Calcium.

Das wasserunlösliche Caseinat kann als solches eingesetzt werden. Es ist darüberhinaus auch möglich, dem Arzneimittelgemisch, enthaltend das lösliche Caseinsalz, physiologisch verträgliche wassserlösliche Salze mehrwertiger Ionen in solchen Mengen zuzusetzen, daß ein Teil des löslichen Caseinats in das unlösliche überführt wird. Bevorzugt werden Chloride, Gluconate, Carbonate, Lactate, Sacchrate u.a., insbesondere Calciumchlorid, Calciumgluconat, Calciumhydrogenphosphat, -lactat, -D-saccharat, -laevulinat oder Mischungen hiervon.

Das Verhältnis von löslichem zu unlöslichem Caseinat liegt **bevorzugte** zwischen 5 und 90 und ganz besonders zwischen 20 und 70%, bezogen auf die Gesamtcaseinat-Menge. Ganz besonders bevorzugt sind Verhältnisse von 30-60%. Die für die Herstellung des unlöslichen Caseinats erforderlichen Mengen an den obengenannten ionischen Verbindungen richten sich nach der Art des Kations, seines Gegenions und dem Molekulargewicht des eingesetzten Caseins und sind vom Fachmann rasch zu bestimmen.

Erfindungsgemäß wird somit überraschenderweise eine kontrollierte biphasische Matrix-kontrollierte Freisetzung erreicht, wobei die die Matrix bildende Substanz kein eingelagertes Fremdprotein enhält.

Als übliche Hilfs- und Zusatzstoffe werden die hierfür in der pharmazeutischen Industrie bekannten Komponenten eingesetzt. Hierzu zählen Tablettierungshilfen, wie hochdisperse Kieselsäure, Magnesiumstearat, mikrokristalline Cellulose, Milchzucker u.a.m.

Als Wirkstoffe können Mittel oder Gemische hiervon für verschiedenste Anwendungsgebiete eingesetzt werden. Hierzu zählen Tranquilizer wie Chlorpromazin und Benzodiazepine, wie z.B. Diazepam; Muskelrelaxantien wie Mephenesin; Antihypertensiva wie alpha-Methyldopa; zentral wirksame Analgetika wie Morphin; peripher wirksame Analgetika wie Paracetamol; nicht-steroidale Antiphlogistika wie Ibuprofen; Lokalanästhetika wie Benzocain; Spasmolytika, wie Papaverin, Prostaglandine wie PEG₂; Antibiotika wie Penicillin, Tetracyclin; das dementielle Syndrom beeinflussende Stoffe wie Memantine; Parkinsontherapeutika wie Amantadin, L-Dopa, Selegilin, Bromocriptin, Metixen; Antimalariastoffe wie Chloroquin; Corticosteroide wie Dexamethason; Androgene wie Methyltestosteron; Östrogene wie Ethinylestradiol; Gestagene wie Levonorgestrel, Sympathomimetika wie Adrenalin; kardiocaskulär wirkende Stoffe wie Nitroglycerin; Diuretika wie Hydrochlorothiazid; Anthelmintika wie Praziquantel; β.Blocker wie Timolol; H₂-Blocker wie Cimetidin; Vitamine wie Ascorbinsäure und andere.

Besonders bevorzugt ist Memantine.

Die erfindungsgemäße, einfach herzustellende Formulierung führt überraschenderweise zu einer kontrollierten 2-stufigen Freisetzung, wobei in der ersten Phase (Freisetzung im Magen) eine dem therapeutischen Ziel angepaßte Menge des Wirkstoffs herausgelöst werden kann.

In der zeiten Phase erfolgt nach einer Verzögerung eine erneute beschleunigte Liberation im Darm, wobei nach einer vorgegebenen Zeit der restliche Wirkstoff freigesetzt werden kann. Auf diese Weise kann eine wirksame Anpassung des jeweiligen Wirkstoffbedarfs erzielt werden, die teilweise Freisetzung führt zu angepaßten Plasmaspiegeln. Lokale Wirkstoffüberkonzentrationen können vermieden werden.

Dieses Liberationsprofil ist weitestgehend unabhängig von den im physiologischen Milieu vorhandenen Enzymen, wie in den nachfolgenden Beispielen aufgezeigt ist, und war mit bisherigen Rezepturen nicht erreichbar.

Ist jedoch in der einen oder anderen oder beiden Liberationsphasen eine veränderte Freisetzung erforderlich, was aufgrund der Art des Wirkstoffs, der Physiognomie des Patienten, der Schwere der Erkrankung erforderlich sein kann, so können der Formulierung Enzyme in geeigneten Mengen, wie z.B. Pepsin und/oder Pankreatin, hinzugesetzt werden. Dabei ändert sich das 2-Phasen-Profil nicht, lediglich die Geschwindigkeit der Liberation innerhalb der Phasen kann leicht variiert werden.

Die Erfindung wird anhand der nachfolgenden Beispiele näher erläutert.

### I. Herstellung der Arzneiformulierungen

### Beispiel 1

| Wirk- und Zusatzstoffe | Gew.-% |
|---|---|
| Memantine-HCl | 20,0 |
| Natriumcaseinat | 46,8 |
| Calciumcaseinat | 31,2 |
| Aerosil® 200 | 1,0 |
| Magnesiumstearat | 1,0 |

Alle Bestandteile außer Magnesiumstearat werden in einem geeigneten Mischer, wie z.B. einen Diosna-Mischer, homogen verteilt, dann Magnesiumstearat zugegeben und durch ein Sieb mit einer mittleren Maschenweite von 300 µm gegeben. Nach einer erneuten Mischung der Preßmasse werden auf einer geeigneten Tablettiermaschine Tabletten mit einer Masse von 100 mg und einem Durchmesser von 6 mm unter Verwendung einer Preßkraft von 8 kN hergestellt.

### Beispiel 2

| Wirk- und Zusatzstoffe | Gew.-% |
|---|---|
| Coffein | 20,0 |
| Natriumcaseinat | 75,0 |
| Calciumchlorid x 2H₂O | 3,0 |
| Aerosil 200® | 1,0 |
| Magnesiumstearat | 1,0 |

Die Bestandteile Coffein und Natriumcaseinat werden in einer geeigneten Wirbelschichtgranulationsapparatur, wie z.B. der eromatic STREA 1, gemischt. Die Granulierung eines 500 g Ansatzes wird mit 400 ml einer wäßrigen Flüssigkeit, die 3 g Calciumchloriddihydrat in gelöster Form enthält, mit einer Pumpgeschwindigkeit von 10 ml/min, einer Einlaßtemperatur von 60°C und einer Auslaßtemperatur von 32°C und einem Zerstäubungsdruck der Zweistoffdüse von ca. 2 bar durchgeführt. Nach einer Nachtrocknung von 10 min bei 60°C und schwacher Zuluft wird das Granulat vom Grobanteil (>1,0 mm) und vom Feinanteil (<0,15 mm) abgetrennt und nach Zumischen von Aerosil und Magnesiumstearat zu Tabletten mit einer Masse von 100 mg und einem Durchmesser von 6 mm mit einer Preßkraft von 8 kN verpreßt.

### Beispiel 3

| Wirk- und Zusatzstoffe | Gew.-% |
|---|---|
| Piroxicam | 20,0 |
| Natriumcaseinat | 65,0 |
| Calciumhydrogenphosphat + 2H₂O | 3,8 |
| Avicel PH 102 | 9,2 |
| Aerosil 200® | 1,0 |
| Magnesiumstearat | 1,0 |

Alle Bestandteile außer Magnesiumstearat werden in einem geeigneten Mischer, wie z.B. einem Diosna-Mischer, homogen verteilt, dann Magnesiumstearat zugegeben und durch ein Sieb mit einer mittleren Maschenweite von 300 µm gegeben. Nach einer erneuten Mischung der Preßmasse werden auf einer geeigneten Tablettiermaschine Tabletten mit einer Masse von 100 mg und einem Durchmesser von 6 mm unter Verwendung einer Preßkraft von 8 kN hergestellt.

### Beispiel 4

| Wirk- und Zusatzstoffe | Gew.-% |
|---|---|
| Ibuprofen | 77,0 |
| Natriumcaseinat | 20,0 |
| Calciumchlorid + 2 H₂O | 1,0 |
| Aerosil 200® | 1,0 |
| Magnesiumstearat | 1,0 |

Von Ibuprofen wird durch Versprühen einer geeigneten Granulierflüssigkeit - wie einer Ethanol/Wasser-Mischung (3+2) - ein Krustengranulat oder einer kolloidhaltigen Granulierflüssigkeit - wie Gelatine/Wasser (1:30) - ein Klebstoffgranulat hergestellt.
Das Granulat wird mit den restlichen Bestandteilen versetzt, in einem geeigneten Mischer gemischt und zu Tabletten mit einer Masse von 520 mg und einem Durchmesser von 12 mm mit einer Preßkraft von 15 kN verpreßt.

### Beispiel 5

| Wirk- und Zusatzstoffe | Gew.-% |
|---|---|
| Memantin-HCl | 20,0 |
| Natriumcaseinat | 48,0 |
| Calciumcaseinat | 32,0 |

Alle Bestandteile werden in einem geeigneten Mischer homogen verteilt und in einem Mischkneter mit einer geeigneten Plastifizierungsflüssigkeit, wie z.B. gereinigtem Wasser oder einer Ethanol/Wasser-Mischung (3+2) in einer Menge von 60%, bezogen auf den Feststoffanteil, innig durchfeuchtet. Die erhaltene Masse wird entweder auf einem Schneckenextruder oder einem Lochwalzenextrukder mit einem Lochscheibendurchmesser von 1,5 mm lyoextrudiert. Die so erhaltenen Stränge werden auf einem Spheronizer zu Pellets mit einem Durchmesser von 1,8 mm zerteilt und ausgerundet. Anschließend werden die Pellets entweder in Kapseln verfüllt oder durch Zumischen von 1% Aerosil und 1% Magnesiumstearat zu Tabletten verpreßt.

### II. Liberationsuntersuchungen

### 1. Verzögerung der Freisetzung eines Wirkstoffs in Abhängigkeit vom Anteil an unlöslichem Caseinat

Folgende Rahmenrezeptur wird verpreßt:

| | |
|---|---|
| Coffein | 20,0 |
| Ca-caseinat | 0-78,0% (bezogen auf Gesamt-Caseinatgehalt) |
| Aerosil 200® | 1,0 |
| Magnesiumstearat | 1,0 |
| NaC | ad 100,0 |

Acht verschiedene Preßmassen mit unterschiedlichem Anteil an Na-Caseinat und Ca-Caseinat (als solches zugesetzt) werden unter Verwendung der Tablettierhilfsmittel Aerosil und Magnesiumstearat rezeptiert. Da keine weiteren Hilfsstoffe zugesetzt werden, wird ein unverfälschtes Bild der Freisetzungseigenschaften gewonnen.

Der Gesamtcaseinatanteil beträgt 78%. Die Pulvermischungen werden auf einer instrumentierten Exacta 1 mit einer eingestellten Geschwindigkeit von 30 Hub/min unter Verwendung von Preßwerkzeugen, 6 mm, flach ohne Facette, mit konstanter Preßkraft von 8 kN zu Tabletten von 100 mg Einzelgewicht komprimiert.

Es werden Produkte mit 0, 10, 20, 30, 40, 50, 70 und 100% Ca-Caseinatanteil erhalten, deren Liberationsprofile bei pH 1,2 (Magen-Milieu-Simulation) bestimmt werden.

Hierbei wurde festgestellt, daß die Freisetzung in diesem Milieu durch den Zusatz an unlöslichem Caseinat zu löslichem Caseinat inhibiert werden kann, wobei je nach Anteil hiervon eine unterschiedliche Verzögerung erhalten wird.

Somit ist ein individuelles Freisetzungsprofil je nach Wirkstoff und Indikationsart möglich.

### 2. Liberationsprofil von mit unterschiedlicher Preßkraft komprimierten Caseinat-Tabletten

Folgende Rezeptur mit einem Ca-caseinat-Anteil vom Gesamtcaseinat von 40% wird nach den obengenannten Bedingungen mit Preßkräften von 4 bis 12 kN zu Tabletten von 100 mg Einzelgewicht komprimiert:

| | |
|---|---|
| Coffein | 20,0 |
| Na-Caseinat | 46,8 |
| Ca-Caseinat | 31,2 |
| Aerosil 200® | 1,0 |
| Magnesiumstearat | 1,0 |

Hierbei zeigt sich, daß unterschiedliche Preßkräfte keinen Einfluß auf das Freisetzungsprofil haben.

### 3. Zusatz von mehrwertigen Salzen zum löslichen Caseinat

Folgende Rahmenrezeptur wird nach oben genannten Bedingungen (siehe II.1 ) hergestellt:

| | |
|---|---|
| Coffein | 20,0 |
| NaC | 65,0 |
| Ca-Salz | X |
| Aerosil® | 1,0 |
| Magnesiumstearat | 1,0 |
| Avicel PH 102® | ad 100 |

Man kann davon ausgehen, daß bei vollständiger Überführung löslicher Caseinate z.B. in das unlösliche Calciumsalz normalerweise ein Calciumgehalt von etwa 1,5% bis 1,7%, gegebenenfalls auch höher, vorliegt, so daß eine erfindungsgemäße Menge leicht ermittelt werden kann.

Folgende pharmazeutisch gebräuchlichen Ca-Salze werden verwendet:

| | In die Rezeptur eingesetzte Menge X |
|---|---|
| Calciumchlorid x 2H₂O | 3,6 |
| Calciumgluconat x H₂O | 10,9 |
| Calciumcarbonat | 2,4 |
| Calciumhydrogenphosphat x 2H₂O | 4,2 |
| Calciumlactat x 5H₂O | 7,5 |
| Calciumnitrat x 4H₂O | 5,8 |
| Calcium-D-saccharat | 6,1 |
| Calciumlaevulinat | 7,5 |

Die Rezepturen werden unter den obengenannten Bedingungen mit konstanter Preßkraft von 8 kN zu Tabletten von 100 mg Einzelgewicht komprimiert.

Hierbei wurde festgestellt, daß alle Calciumsalze ein fast gleiches Liberationsprofil ergeben. Die t₅₀-Werte schwanken in einem engen Bereich zwischen 100 und 113 min, die t₉₀-Werte zwischen 262 und 295 min.

Gleiche Ergebnisse erhält man auch mit anderen mehrwertigen Ionen.

### 4. Einfluß des Inkubationsmediums

Verschiedene nach II.1 hergestellte Komprimate mit unterschiedlichem Anteil an unlöslichem Caseinat (Calciumcaseinat) werden nachfolgend bezüglich ihres Liberationsprofils bei wechselndem Medium mit/ohne Enzym untersucht.

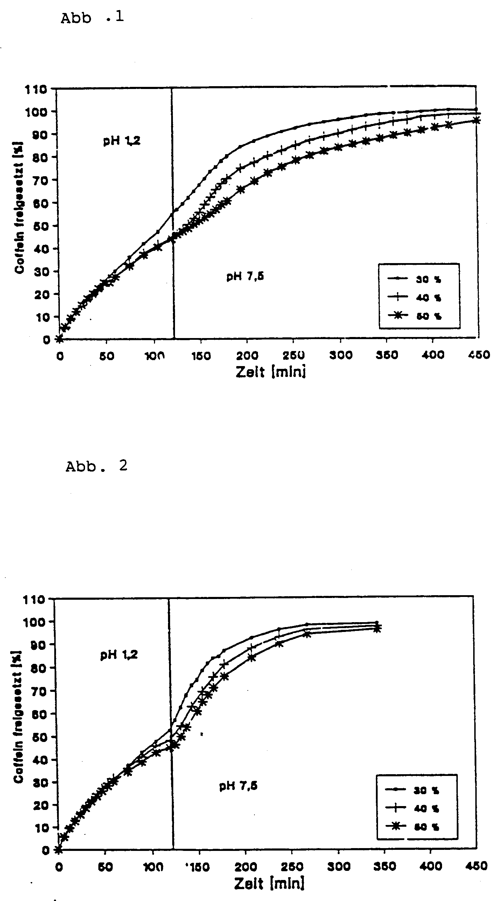

Die Ergebnisse sind in den Abb. 1 und 2 dargestellt.

Diese Abbildungen zeigen die biphasischen Verläufe der erfindungsgemäß hergestellten Rezepturen mit 30, 40 und 50% Ca-Caseinat-Anteil. Die Ergebnisse sind in nachfolgender Tabelle 1 dargestellt (Mediumwechsel nach 2 Stunden).

**Tabelle 1**

| | t₅₀[min] | | | t₉₀[min] | | |
|---|---|---|---|---|---|---|
| pH/Ca-Caseinat [%] | 30 | 40 | 50 | 30 | 40 | 50 |
| 1,2/7,5 ohne Enzym (Abb.1) | 111 | 138 | 143 | 232 | 300 | 376 |
| 1,2/7,5 mit Enzym (Abb.2) | 112 | 123 | 132 | 195 | 220 | 239 |

Überraschenderweise zeigte sich, daß das gewünschte Liberationsprofil relativ unabhängig von vorhandenem Enzym ist. Die geringe enzyminduzierte Beschleunigung garantiert dabei letztendlich eine 100%ige Freisetzung in der 2. Phase nach etwa 5 Stunden.

### 5. Freisetzung von Memantine aus erfindungsgemäß hergestellten Tabletten

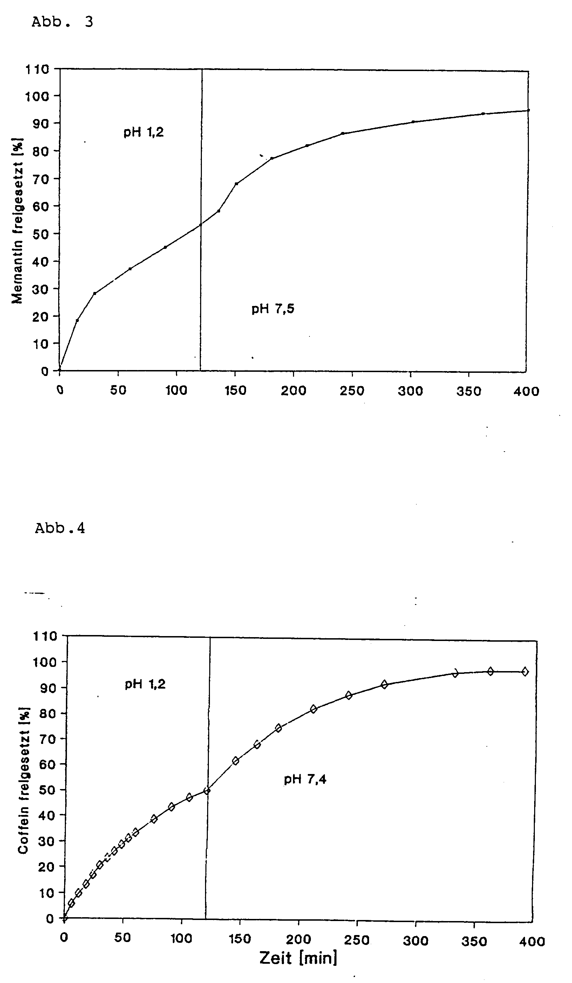

Eine nach Beispiel I.1. hergestellte Rezeptur wird wie oben bei pH 1,2/pH 7,5 (ohne Enzym, Mediumwechsel nach 2 Stunden) untersucht. Das zweistufige Liberationsprofil ist in Abb. 3 dargestellt.

### 6. Freisetzung von Coffein aus einer erfindungsgemäß hergestellten Rezeptur

Eine nach Beispiel I.2. hergestellte Formulierung wird wie oben bei pH 1,2/pH 7,4 (ohne Enzym, Mediumwechsel nach 2 Stunden) untersucht. Auch hier ergibt sich ein zweistufiges Liberationsprofil, das in Abb. 4 dargestellt ist.

Mit den erfindungsgemäßen Zusammensetzungen lassen sich somit auf einfache Weise aus bekannten physiologisch verträglichen Komponenten Formulierungen mit optimal angepaßtem Freisetzungsprofil herstellen.

## Patentansprüche

1. Verfahren zur Herstellung von festen Arzneiformkörpern mit Matrix-kontrollierter protrahierter 2-Stufen-Freisetzung durch Kompression, Granulierung, Extrusion, Pelletisierung und/oder Tablettierung auf trockenem oder feuchtem Weg von Mischungen, enthaltend einen oder mehrere Wirkstoffe sowie übliche Hilfs- und Zusatzstoffe, dadurch gekennzeichnet, daß man der Mischung insgesamt 5-98%, bezogen auf die Gesamtmenge, eines wasserlöslichen und eines wasserunlöslichen Salzes von Casein zusetzt, oder daß man ein wasserlösliches Caseinat zusetzt und Salze zwei- oder mehrwertiger Kationen zur Bildung des unlöslichen Caseinats zufügt, so daß der Gesamtcaseinatgehalt 5-98% beträgt, wobei die Caseinsalzmischung 5-95% des wasserunlöslichen Caseinsalzes aufweist.

2. Verfahren gemäß Anspruch 1, dadurch gekennzeichnet, daß man 10-90% der Caseinsalzmischung zusetzt.

3. Verfahren gemäß Anspruch 1, dadurch gekennzeichnet, daß man 30-80% der Caseinsalzmischung zusetzt.

4. Verfahren gemäß einem der Ansprüche 1 bis 3, dadurch gekennzeichnet, daß man eine Caseinsalzmischung, enthaltend 20-70%, des wasserunlöslichen Caseinsalzes, bezogen auf den Gesamtcaseingehalt, zusetzt.

5. Verfahren gemäß einem der Ansprüche 1 bis 4, dadurch gekennzeichnet, daß man als wasserunlösliches Caseinsalz Calciumcaseinat verwendet.

6. Verfahren gemäß einem der Ansprüche 1-3, dadurch gekennzeichnet, daß man dem wasserlöslichen Caseinat zwei- oder mehrwertige Kationen in einer Menge zufügt, daß das Verhältnis von wasserlöslichem zu entstehendem wasserunöslichen Caseinsalz 5 bis 90%, vorzugsweise 20 bis 70%, beträgt.

7. Verfahren nach Anspruch 6, dadurch gekennzeichnet, daß man Calciumionen zusetzt.

8. Verfahren gemäß einem der Ansprüche 1 bis 7, dadurch gekennzeichnet, daß man als wasserlösliches Caseinsalz Natriumcaseinat verwendet.

9. Verfahren gemäß einem der Ansprüche 1 bis 8, dadurch gekennzeichnet, daß man der Mischung zusätzlich Enzyme zusetzt.

10. Verfahren gemäß Anspruch 9, dadurch gekennzeichnet, daß man als Enzym Pankreatin und/oder Pepsin verwendet.

11. Verfahren gemäß einem der Ansprüche 1 bis 10, dadurch gekennzeichnet, daß man als Wirkstoff Memantine verwendet.

## Claims

1. Process for the production of solid pharmaceutical dosage forms with matrix-controlled, prolonged 2-stage release, by the compression, granulation, extrusion, pelleting and/or tableting, by the dry or wet method, of mixtures comprising one or more active ingredients together with conventional auxiliary substances and additives, characterized in that a total of 5-98%, based on the total amount, of a water-soluble and a water-insoluble casein salt is added to the mixture, or in that a water-soluble caseinate is added and salts of divalent or polyvalent cations are incorporated to form the insoluble caseinate, so that the total caseinate content is 5-98%, the casein salt mixture containing 5-95% of the water-insoluble casein salt.

2. Process according to Claim 1, characterized in that 10-90% of the casein salt mixture is added.

3. Process according to Claim 1, characterized in that 30-80% of the casein salt mixture is added.

4. Process according to one of Claims 1 to 3, characterized in that a casein salt mixture containing 20-70% of the water-insoluble casein salt, based on the total casein content, is added.

5. Process according to one of Claims 1 to 4, characterized in that the water-insoluble casein salt used is calcium caseinate.

6. Process according to one of Claims 1-3, characterized in that divalent or polyvalent cations are incorporated into the water-soluble caseinate in an amount such that the ratio of water-soluble casein salt to water-insoluble casein salt being formed is 5 to 90%, preferably 20 to 70%.

7. Process according to Claim 6, characterized in that calcium ions are added.

8. Process according to one of Claims 1 to 7, characterized in that the water-soluble casein salt used is sodium caseinate.

9. Process according to one of Claims 1 to 8, characterized in that enzymes are also added to the mixture.

10. Process according to Claim 9, characterized in that the enzyme used is pancreatin and/or pepsin.

11. Process according to one of Claims 1 to 10, characterized in that the active ingredient used is memantine.

## Revendications

1. Procédé de préparation de corps façonnés médicamenteux solides à libération prolongée en des stades, contrôlée par une matrice, par compression, granulation, extrusion, pastillage et/ou fabrication de comprimés par voie sèche ou humide de mélanges contenant une ou plusieurs substances actives, ainsi que des agents auxiliaires et des additifs classiques, caractérisé en ce que l'on ajoute au mélange 5 à 98 % en tout, par rapport à la quantité totale, d'un sel de caséine soluble dans l'eau et d'un sel de caséine insoluble dans l'eau, ou en ce que l'on ajoute un caséinate soluble dans l'eau et on introduit des sels de cations divalents ou polyvalents destinés à former le caséinate insoluble, de façon que la teneur totale a caséinates soit de 5 à 98 %, le mélange de sels de caséine contenant 5 à 95 % du sel de caséine insoluble dans l'eau.

2. Procédé selon la revendication 1, caractérisé en ce que l'on ajoute 10 à 90 % du mélange de sels de caséine.

3. Procédé selon la revendication 1, caractérisé en ce que l'on ajoute 30 à 80 % du mélange de sels de caséine.

4. Procédé selon l'une des revendications 1 à 3, caractérisé en ce que l'on ajoute un mélange de sels de caséine contenant 20 à 70 % du sel de caséine insoluble dans l'eau par rapport à la teneur totale en caséine.

5. Procédé selon l'une des revendications 1 à 4, caractérisé en ce que l'on utilise du caséinate de calcium comme sel de caséine insoluble dans l'eau.

6. Procédé selon l'une des revendications 1 à 3, caractérisé en ce que l'on ajoute au caséinate soluble dans l'eau des cations divalents ou polyvalents en une quantité telle que le rapport du sel de caséine soluble dans l'eau au sel de caséine insoluble dans l'eau qui va se former est compris entre 5 et 90 %, de préférence entre 20 et 70 %.

7. Procédé selon la revendication 6, caractérisé en ce que l'on ajoute des ions calcium.

8. Procédé selon l'une des revendications 1 à 7, caractérisé en ce que l'on utilise du caséinate de sodium comme sel de caséine soluble dans l'eau.

9. Procédé selon l'une des revendications 1 à 8, caractérisé en ce que l'on ajoute en outre des enzymes au mélange.

10. Procédé selon la revendication 9, caractérisé en ce que l'on utilise comme enzyme de la pancréatine et/ou de la pepsine.

11. Procédé selon l'une des revendications 1 à 10, caractérisé en ce que l'on utilise de la mémantine comme substance active.
